# EUROPEAN PATENT APPLICATION

(11) **EP 4 417 209 A1**
(43) Date of publication of application: **21.08.2024**
(21) Application number: 22879977.1
(22) Date of filing: 16.08.2022
(51) Int. Cl.: A61K 33/24, A61K 9/51, A61K 47/26, A61K 47/32, A61K 47/34, A61K 47/36, A61K 49/00, A61P 35/00, B82Y 5/00, B82Y 40/00

(54) **TANTALUM NANO-COMPLEX, AND PREPARATION METHOD THEREFOR AND USE THEREOF, LYMPHATIC TRACER, AND RADIOTHERAPY SENSITIZER**

(30) Priority: 15.10.2021 CN 202111203003
(71) Applicant: Institute Of High Energy Physics Chinese Academy of Sciences, Beijing 100143 (CN)
(72) Inventor: GU, Zhanjun, Beijing 100143 (CN); JI, Chao, Beijing 100143 (CN); ZHAO, Maoru, Beijing 100143 (CN); DONG, Xinghua, Beijing 100143 (CN); ZHU, Shuang, Beijing 100143 (CN)
(74) Representative: Zaboliene, Reda
(86) International application number: PCT/CN2022/112635
(87) International publication number: WO 2023/061030

(57) **Abstract**

The invention relates to a tantalum nanocomposite and a preparation method and application thereof, lymph tracer and radiosensitizer. The tantalum nanocomposite provided in the invention includes tantalum nanoparticle and bio-surfactant acting on the tantalum nanoparticle. The tantalum nanocomposite provided in the invention has good biosafety and can improve the effect of radiation therapy as a radiosensitizer.

## Description

The application requires the priority of the Chinese patent application filed with the Chinese Patent Office with the application number CN202111203003.1 and the invention name "tantalum nanocomposite and lymph tracer and radiosensitizer containing the tantalum nanocomposite" on October 15, 2021, all of which are incorporated in this application by citation.

### Technical field

The invention relates to the technical field of tantalum nanomaterial, in particular to a tantalum nanocomposite and a preparation method and application thereof, lymph tracer and radiosensitizer.

### Background technology

A malignant tumor is a disease threatening life in the world, and lymph circulation is a major approach for tumor metastasis. Sentinel lymph node is the first lymph node receiving the lymph circulation in the tumor area earliest and the first to have tumor metastasis. If there is no tumor metastasis in the sentinel lymph nodes, there is a little chance of tumor metastasis in other lymph nodes in the area; however, the lymph node in tumor area hides in connective tissue generally and is difficult to be observed directly; therefore, the lymph node needs to be stained and traced to identify the lymph node with cancerometastasis and is removed thoroughly.

In addition, radiation therapy is one of the treatments to inhibit tumor growth. Due to low doses of X-ray are not effective in inhibiting tumor and high doses of X-ray will increase the damage to normal tissue, whereby limiting the effect of radiation therapy.

Therefore, a material with good bio-compatibility used as lymph tracer and/or radiosensitizer is expected in the field.

### Summary of the invention

In view of the above analysis, an embodiment of the invention aims to provide a tantalum nanocomposite to improve the effect of radiation therapy.

On one hand, a tantalum nanocomposite is provided in an embodiment of the invention, including a tantalum nanoparticle and a bio-surfactant acting on the tantalum nanoparticle.

According to an embodiment of the invention, the bio-surfactant includes a polymer surfactant.

According to an embodiment of the invention, the polymer surfactant includes a polyvinyl pyrrolidone, a tween 20, a polyactic acid, one or more of chitosan.

According to an embodiment of the invention, the particle diameter of the tantalum nanoparticle is 70-200nm.

According to an embodiment of the invention, the preparation method of the tantalum nanocomposite includes the following steps:
providing a mixture containing the tantalum nanoparticle, the bio-surfactant and water; and performing ball-milling on the mixture.

According to an embodiment of the invention, the concentration of the bio-surfactant in water is 0.2-1g/mL; and/or,
the mass ratio of the sum of the mass of the bio-surfactant and the water to the tantalum nanoparticle is 1:1-5:1.

According to an embodiment of the invention, the rotational speed of the ball-milling is 100-300rpm, and the time duration is 1-3h.

According to an embodiment of the invention, the preparation method further includes centrifuging the mixture after performing ball-milling.

On the other hand, an embodiment of the invention provides a lymph tracer, including the tantalum nanocomposite of any of the above.

An embodiment of the invention further provides a radiosensitizer, including the tantalum nanocomposite of any of the above.

An embodiment of the invention further provides the application of the tantalum nanocomposite of any of the above as the lymph tracer and/or radiosensitizer .

Compared with the prior art, at least one of the following beneficial effects can be achieved with the invention:
1. the tantalum nanocomposite in an embodiment of the invention has good biosafety and can improve the effect of radiation therapy as radiosensitizer;
2. the tantalum nanocomposite in an embodiment of the invention can stain the lymph node, so as to trace the lymph node and help removing the lymph node.

In the invention, the above technical solutions can also be combined with each other to achieve more optimal combination solutions. Other characteristics and advantages will be stated in the following specification, and, part of the advantages are obvious in the specification or understood through implementing the invention. The objective and other advantages of the invention can be achieved and obtained from the contents indicated specifically in the specification and the attached drawings.

### Description of attached drawings

The attached drawings are only used to show the embodiments, not to limit the invention. Wherein:
Fig. 1 is a physical image of the tantalum nanocomposite dispersion solution of Example 1;
Fig. 2 is an image of the tantalum nanocomposite of Example 1 under a transmission electron microscope;
Fig. 3 is an X-ray diffraction spectrum of the tantalum nanocomposite of Example 1;
Fig. 4 is a degradation rate diagram of rhodamine B by the tantalum nanocomposite obtained from Example 1-4;
Fig. 5a is a cell cloning image of contrast group 1 in Example 1;
Fig. 5b is a cell cloning image of example group 1-1 in Example 1;
Fig. 5c is a cell cloning image of contrast group 2 in Example 1;
Fig. 5d is a cell cloning image of example group 1-2 in Example 1;
Fig. 6a is an image for lymph node of the foot pads of mice before injecting tantalum nanocomposite dispersion in Example 1;
Fig. 6b is an image for lymph node of the foot pads of mice after injecting tantalum nanocomposite dispersion in Example 1.

### Specific embodiments

The preferable embodiments of the invention are described specifically below, wherein the drawings are the part of the invention and are used to state the principle of the invention combined with the embodiments of the invention, not to limit the scope of the invention.

A tantalum nanocomposite or tantalum nanomaterial is provided in an embodiment of the invention, including a tantalum nanoparticle and a bio-surfactant acting on the tantalum nanoparticle.

In the tantalum nanocomposite of an embodiment of the invention, on one hand, tantalum has good bio-compatibility and higher high-energy ray energy deposition capacity, and can be used as radiosensitizer to improve biosafety and effects of radiation therapy; specifically, after tantalum nanocomposite is added to radiation therapy, the same doses of X-ray cause more cells damage, which shows the enhanced effect.

On the other hand, bio-surfactant is used to modify tantalum nanoparticle and coated on the surface of tantalum nanoparticle, improving the disperstiveness of tantalum nanocomposite in water and is beneficial to the use of tantalum nanocomposite as radiosensitizer.

In an embodiment, the particle diameter of the tantalum nanoparticle can be 70-200nm, such as: 70nm, 80nm, 90nm, 100nm, 150nm, 180nm, 200nm, and etc..

In an embodiment, the bio-surfactant may be a degradable polymer, and has the advantages of non-toxic, biodegradable, ecological safety and high surface activity. The bio-surfactant has many different kinds, specifically, including glycolipid, lipopeptide and lipoprotein, fatty acid and phospholipid and neutral lipid and polymer surfactant.

In an embodiment, the bio-surfactant has good bio-compatibility and may be polymer surfactant, such as polyvinyl pyrrolidone, tween 20, polyactic acid and one or more of chitosan.

In an embodiment, the mass average molar mass of the polyvinyl pyrrolidone may be 58,000.

The tantalum nanocomposite in an embodiment of the invention can be prepared through physisorption.

An embodiment of the invention further provides a preparation method of the tantalum nanocomposite, including the following steps:
mixing the bio-surfactant with water to obtain a first mixture;
mixing the tantalum nanoparticle (tantalum powder) with the first mixture to obtain a second mixture, which is processed through ball-milling to enable bio-surfactant to be adsorbed on the surface of tantalum nanoparticle, whereby a tantalum nanocomposite composed of bio-surfactant and tantalum nanoparticle is formed; and
centrifuging the above system containing tantalum nanocomposite to remove the free bio-surfactant and obtain the tantalum nanocomposite.

In an embodiment, the water used to prepare tantalum nanocomposite may be ultrapure water.

In an embodiment, the first mixture may be aqueous solution of polyvinyl pyrrolidone at a concentration of 0.2-1g/mL, such as 0.5g/mL, 0.6g/mL, 0.8g/mL and 1g/mL.

In an embodiment, the mass ratio of the tantalum powder and the first mixture (e.g. aqueous solution of polyvinyl pyrrolidone) is 1:1-1:5, such as 1:1, 1:2, 1:3, 1:4 and 1:5.

In an embodiment, the rotational speed of the ball-milling may be 100rpm-300rpm, such as 100rpm, 150rpm, 200rpm, 250rpm and 300rpm; the time duration of the ball-milling may be 1-3h, such as 1.5h, 2h, 2.5h and 3h.

In an embodiment, the rotational speed of centrifugation may be 12,000rpm and the time duration may be 10min.

An embodiment of the invention further provides a lymph tracer, including the tantalum nanocomposite above.

An embodiment of the invention further provides a radiosensitizer, including the tantalum nanocomposite above.

The tantalum nanocomposite in the embodiment of the invention can enhance the X-ray energy deposition and effectively enhance the production of free radicals and can be used as a radiosensitizer.

The tantalum nanocomposite in the embodiment of the invention has lymphatic staining tracing properties and can be used as a lymph tracer. Specifically, when using, tantalum nanocomposite can be dispersed into water to form dispersion solution and to be injected into a living organism (e.g. the human body) directly. After the injection, the liquid containing tantalum nanocomposite flows towards and stains the lymph node, whereby the lymph node can be traced and the lymph node can be removed easily, which can be used in lymph node removal surgery.

The tantalum nanocomposite of the embodiment of the invention has good biosafety and can be used as lymph tracer and radiosensitizer at the same time, the integration of the lymphatic tracing and radiosensitization can be achieved, which has a broad application prospect in the fields such as nanomedicine and tumor inhibition.

Further description of the preparation and application of tantalum nanocomposite in an embodiment of the invention is stated in combination with the attached drawings and specific examples in the following. Wherein, the raw materials used in the example can be purchased at market. The tantalum powder used is purchased from Xuzhou Jie Innovative Materials Technology Co., Ltd.. The average particle diameter is 70nm and the mass average molar mass of polyvinyl pyrrolidone, polylactic acid and chitosan is 58,000, 60,000 and 3,200 respectively.

### Example 1

The preparation of tantalum nanocomposite
(1) Dissolving the polyvinyl pyrrolidone in the ultrapure water to obtain an aqueous solution of polyvinyl pyrrolidone at a concentration of 1g/mL.
(2) Mixing the tantalum powder with the aqueous solution of polyvinyl pyrrolidone obtained in the step (1) with a mass ratio of 1:5. Performing ball-milling on the obtained mixture, and the rotational speed of the ball-milling is 300rpm and the time duration is 3h.
(3) Centrifuging the mixed liquor after performing ball-milling, and the rotational speed of centrifugation is 12,000rpm and the time duration is 10min. Dispersing the centrifugal products into the ultrapure water to obtain the dispersion liquid of tantalum nanomaterial (tantalum nanocomposite) modified by the polyvinyl pyrrolidone.

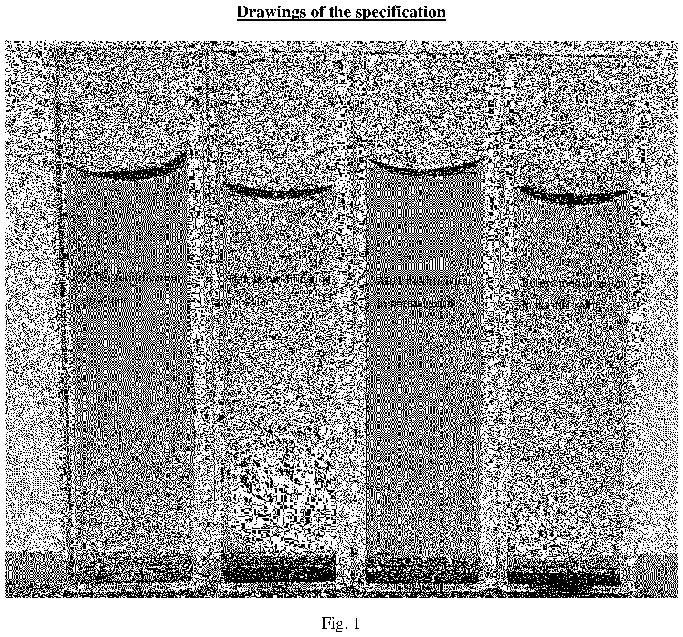

Fig. 1 is the image of the dispersion of raw tantalum powder in water and normal saline before and after modifying by polyvinyl pyrrolidone. It can be seen from Fig. 1, the disperstiveness in water of the tantalum nanocomposite is much better than that of tantalum nanoparticle, which fully indicates that in the prepared tantalum nanocomposite, the polyvinyl pyrrolidone acts on the surface of the tantalum nanoparticle, thereby improving the dispersiveness of the tantalum nanoparticle.

The transmission electron microscopy characterization on tantalum nanocomposite

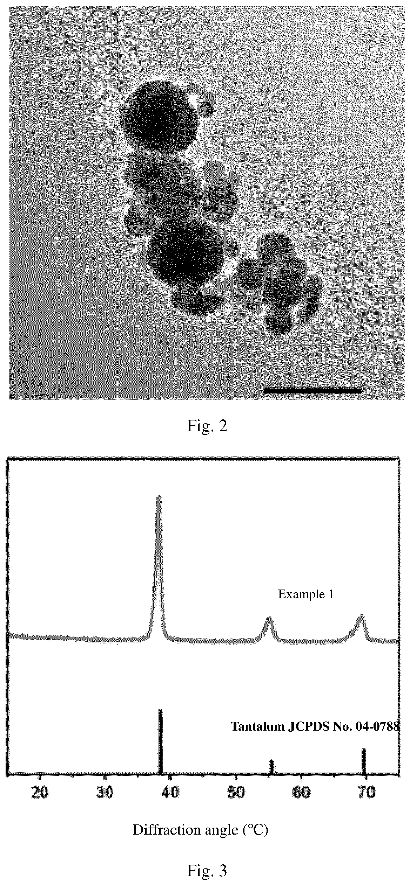

Performing transmission electron microscopy characterization on the obtained tantalum nanocomposite above, the images obtained from electron microscope are shown in Fig. 2. It can be seen from Fig. 2, the particle diameter of the nanoparticle of the tantalum nanocomposite is about 70nm, which indicates the modification by polyvinyl pyrrolidone has no substantial impact on the size of the tantalum nanoparticle.

The X-ray diffraction characterization on tantalum nanocomposite

Performing X-ray diffraction characterization on the obtained tantalum nanocomposite above, and the resulting spectrum is shown in Fig. 3. It can be seen from Fig. 3, compared with the standard JCPDS spectrum, there is no other miscellaneous peaks in Example 1, which indicates the tantalum powder used is the tantalum nanomaterial with very high purity and almost has no other impurities.

The characterization on free radical generating capacity of tantalum nanocomposite

Rhodamine B is a typical model to study the free radical generating in vitro through degradation and has a characteristic peak in the position of 554nm. When rhodamine B reacts with the free radical, the absorption value of the characteristic peak is reduced, through which, the effect of the free radical generating can be evaluated. The capacity of the tantalum nanocomposite to generate free radicals is evaluated based on the principle in the following.

Contrast group: irradiating the rhodamine B solution at a concentration of 5µg/mL for 10min through an X-ray tube (50kV, 75µA).

Example groups 1-4: irradiating the solution containing 5µg/mL rhodamine B and 250µg/mL tantalum nanocomposite obtained in Examples 1-4 for 10min through an X-ray tube (50kV, 75µA) respectively.

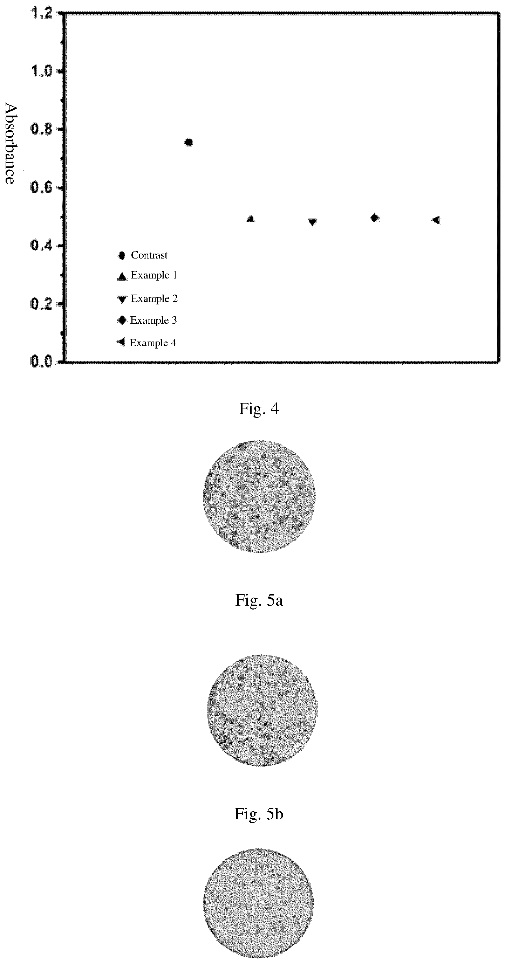

Fig. 4 shows the absorbance of the above irradiation, and it can be seen that the absorbance of the contrast group is close to 0.8, and the absorbance of example groups 1-4 is about 0.5. The result shows that after adding the tantalum nanocomposite, the absorption value of the characteristic peak of rhodamine B is reduced, which further indicates the tantalum nanocomposite has good free radical generating capacity under the X ray excitation.

Determination on radio sensitization capacity of tantalum nanocomposite

Cell clone is a method to determine cell proliferation ability, the effect of the radiosensitization of the tantalum nanocomposite is stated through evaluating the effect of different groups on cell cloning experiments.

Culturing the breast cancer tumor cells in six-well plate with 1,000 cells for each well, replacing the complete medium solution into the six-well plate after 24h, and fixing with paraformaldehyde and staining with Giemsa after 7 days; the system obtained is named as contrast group 1, and the image is shown in Fig. 5a.

Culturing the breast cancer tumor cells in a six-well plate with 1,000 cells for each well, replacing the complete culture medium solution prepared above at a concentration of 250µg/mL of the tantalum nanocomposite modified by the polyvinyl pyrrolidone into the six-well plate after 24h and fixing with paraformaldehyde and staining with Giemsa after 7 days; the system obtained is named as example group 1-1, and the image is shown in Fig. 5b.

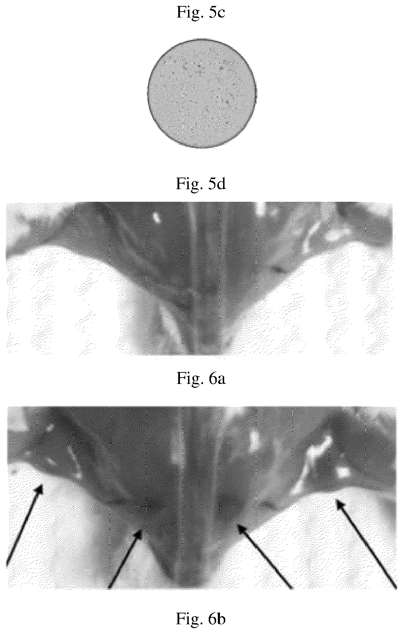

Culturing the breast cancer tumor cells in a six-well plate with 1,000 cells for each well, replacing the complete medium solution into the six-well plate and irradiating with 6Gy X-ray after 24h, and fixing with paraformaldehyde and staining with Giemsa; the system obtained is named as contrast group 2, and the image is shown in Fig. 5c.

Culturing the breast cancer tumor cells in a six-well plate with 1,000 cells for each well, replacing the complete culture medium solution prepared above at a concentration of 250µg/mL of the tantalum nanocomposite modified by the polyvinyl pyrrolidone into the six-well plate and irradiating with 6Gy X-ray after 24h, and fixing with paraformaldehyde and staining with Giemsa after 7 days; the system obtained is named as example group 1-2, and the image is shown in Fig. 5d.

In can be seen from Figs. 5a to 5d, both contrast group 1 (Fig. 5a) and example group 1-1 (Fig. 5b) without X-ray irradiation have more cell clones and fewer cell clones in contrast group 2 with X-ray irradiation, while example groups 1-2 containing tantalum nanocomposite are able to form fewer cell clones under X-ray (6Gy) irradiation than the group (contrast group 2) with only X-ray irradiation, which shows that tantalum nanocomposite has good ability of radiosensitization.

Determination on lymphatic tracing ability of tantalum nanocomposite

Injecting the above aqueous dispersion (75mg/mL,75µL) of the tantalum nanocomposite into the foot pads of BALB/c male mice, observing the staining of the lymph node after 90 minutes and killing the mice for taking out the lymph node to be observed. Fig. 6a is the image of lymph node of the mice before injecting aqueous dispersion of tantalum nanocomposite, and Fig. 6b is the image after injection; the color change of the area shown by the arrow in Fig. 6b before and after injection shows that both popliteal lymph node and nodi lymphatici iliaci have a better staining effect, which further shows that tantalum nanocomposite has the ability of lymphatic staining and tracing.

### Example 2

The same raw materials and steps with Example 1 are adopted in the example, the only difference is that: the bio-surfactant adopted is tween 20. The result of the characterization on free radical generating capacity of the tantalum nanocomposite is shown in Fig. 4.

### Example 3

The same raw materials and steps with Example 1 are adopted in the example, the only difference is that: the bio-surfactant adopted is polylactic acid. The result of the characterization on free radical generating capacity of the tantalum nanocomposite is shown in Fig. 4.

### Example 4

The same raw materials and steps with Example 1 are adopted in the example, the only difference is that: the bio-surfactant adopted is chitosan. The result of the characterization on free radical generating capacity of the tantalum nanocomposite is shown in Fig. 4.

The above results fully indicate that the tantalum nanocomposite in the examples of the invention can enhance the generation of free radicals in tumor area under X-ray irradiation to improve the effect of radiation therapy. In addition, the tantalum nanocomposite in the examples of the invention can stain and trace the lymph node, which helps to remove the lymph node.

The above mentioned are only the better embodiments of the invention, not the limitation of the invention. Within the technical scope disclosed by the invention, any modifications or replacements that can easily be thought of by any skilled familiar with the technical field shall be covered by the protection scope of the invention.

## Claims

1. A tantalum nanocomposite, comprising a tantalum nanoparticle and a bio-surfactant coated on the surface of the tantalum nanoparticle.

2. The tantalum nanocomposite according to claim 1, wherein the bio-surfactant comprises a polymer surfactant.

3. The tantalum nanocomposite according to claim 2, wherein the polymer surfactant comprises one or more of polyvinyl pyrrolidone, Tween 20, polyactic acid and chitosan.

4. The tantalum nanocomposite according to claim 3, wherein the polyvinyl pyrrolidone has a mass average molar mass of 58,000, the polylactic acid has a mass average molar mass of 60,000, and the chitosan has a mass average molar mass of 3,200.

5. The tantalum nanocomposite according to claim 1, wherein the bio-surfactant comprises a glycolipid, a lipopeptide, a lipoprotein, a fatty acid, a phospholipid and a neutral lipid.

6. The tantalum nanocomposite according to any one of claims 1-5, wherein the tantalum nanoparticle has a particle diameter of 70-200 nm.

7. A preparation method for the tantalum nanocomposite of any one of claims 1-6, comprising the following steps of:
providing a mixture which contains the tantalum nanoparticle, the bio-surfactant and water, and
performing ball-milling treatment on the mixture to obtain the tantalum nanocomposite.

8. The preparation method according to claim 7, wherein the bio-surfactant has a concentration of 0.2-1 g/mL in water; and/or,
a mass ratio of the sum of the bio-surfactant and the water by mass to the tantalum nanoparticle is 1:1-5:1.

9. The preparation method according to claim 7, wherein the step of providing a mixture is as follows:
dissolving the bio-surfactant into water to obtain a bio-surfactant aqueous solution, and
mixing the bio-surfactant aqueous solution with the tantalum nanoparticle.

10. The preparation method according to claim 7, wherein the ball-milling treatment is performed at a rotational speed of 100-300 rpm for 1-3 h.

11. The preparation method according to claim 10, further comprising a step of centrifuging the ball-milled mixture at the end of the ball-milling treatment.

12. The preparation method according to claim 11, wherein the centrifugation is performed at a rotational speed of 12,000 rpm for 10 min.

13. An application of the tantalum nanocomposite of any one of claims 1-6 or the tantalum nanocomposite prepared by the method of any one of claims of 7-12 in lymphatic tracing and/or radio sensitization.

14. The application according to claim 13, wherein the tantalum nanocomposite is used as a lymphatic tracer and/or a radiosensitizer.

15. A lymphatic tracer, comprising the tantalum nanocomposite of any one of claims 1-6 or the tantalum nanocomposite prepared by the method of any one of claims 7-12.

16. A radiosensitizer, comprising the tantalum nanocomposite of any one of claims 1-6 or the tantalum nanocomposite prepared by the method of any one of claims 7-12.
